# EUROPÄISCHE PATENTSCHRIFT

(11) **EP 2 533 641 B1**
(45) Veröffentlichungstag und Bekanntmachung des Hinweises auf die Patenterteilung: **13.07.2016**
(21) Anmeldenummer: 11701840.8
(22) Anmeldetag: 04.02.2011
(51) Int. Cl.: A01N 43/713, C07D 401/14, C07D 403/06, A61K 31/4439, A61P 33/00

(54) **HYDRAZIN-SUBSTITUIERTE ANTHRANILSÄUREDERIVATE**
HYDRAZINE-SUBSTITUTED ANTHRANILIC ACID DERIVATIVES
DÉRIVÉS D'ACIDES D'ANTHRANILE SUBSTITUÉS PAR HYDRAZINE

(30) Priorität: 09.02.2010 US 302651 P; 09.02.2010 EP 10153013
(43) Veröffentlichungstag der Anmeldung: 19.12.2012
(73) Patentinhaber: Bayer CropScience AG, 40789 Monheim am Rhein (DE)
(72) Erfinder: FISCHER, Rüdiger, 50259 Pulheim (DE); WROBLOWSKY, Heinz-Juergen, 40764 Langenfeld (DE); GESING, Ernst Rudolf, 40699 Erkrath (DE); GRONDAL, Christoph, 50937 Köln (DE); HENSE, Achim, 51379 Leverkusen (DE); VOERSTE, Arnd, 50674 Köln (DE)
(74) Vertreter: BIP Patents
(86) Internationale Anmeldenummer: PCT/EP2011/051665
(87) Internationale Veröffentlichungsnummer: WO 2011/098408

(56) Entgegenhaltungen:
- WO-A1-2007/043677
- WO-A1-2007/144100

## Beschreibung

Die vorliegende Erfindung betrifft neue Hydrazin-substituierte Anthranilsäurederivate, deren Anwendung als Insektizide und Akarizide zur Bekämpfung tierischer Schädlinge, auch in Kombination mit weiteren Mitteln zur Wirkungssteigerung, und mehrere Verfahren zu ihrer Herstellung.

Anthranilsäurederivate mit insektiziden Eigenschaften sind in der Literatur bereits beschrieben, z.B. in WO 01/70671, WO 03/015519, WO 03/016284, WO 03/015518, WO 03/024222, WO 03/016282, WO 03/016283, WO 03/062226, WO 03/027099, WO 04/027042, WO 04/033468, WO 2004/046129, WO 2004/067528, WO 2005/118552, WO 2005/077934, WO 2005/085234, WO 2006/023783, WO 2006/000336, WO 2006/040113, WO 2006/111341, WO 2007/006670, WO 2007/024833, WO2007/020877, WO 2007/144100, WO2007/043677, WO2008/126889, WO2008/126890, WO2008/126933

Die gemäß den oben genannten Schriften bereits bekannten Wirkstoffe weisen aber in ihrer Anwendung teils Nachteile auf, sei es, dass sie nur eine geringe Anwendungsbreite aufweisen, sei es, dass sie keine zufriedenstellende insektizide oder akarizide Wirkung aufweisen.

Es wurden nun neue Hydrazin-substituierte Anthranilsäurederivate gefunden, welche gegenüber den bereits bekannten Verbindungen Vorteile aufweisen, z.B. seien bessere biologische oder ökologische Eigenschaften, breitere Anwendungsmethoden, eine bessere insektizide, akarizide Wirkung, sowie eine gute Verträglichkeit gegenüber Nutzpflanzen beispielhaft genannt. Die Hydrazin-substituierten Anthranilsäurederivate können in Kombination mit weiteren Mitteln zur Verbesserung der Wirksamkeit insbesondere gegen schwierig zu bekämpfende Insekten eingesetzt werden.

Gegenstand der vorliegenden Erfindung sind daher neue Hydrazin-substituierte Anthranilsäurederivate der Formel (I)

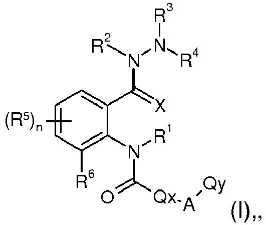

Die erfindungsgemäßen Verbindungen sind durch die Formel (I) allgemein definiert. Bevorzugt, besonders bevorzugt und ganz besonders sind Verbindungen der Formel (I), in welcher
- R¹: für Wasserstoff steht,
- R², R³: unabhängig voneinander für Wasserstoff, Methyl, Ethyl, iso-Propyl, tert-Butyl stehen,
- R⁴: für -C(=O)-R⁸, -C(=O)-OR⁹ steht,
- R⁵: für Wasserstoff, Methyl, Trifluormethyl, Cyano, Fluor, Chlor, Brom, Iod oder Trifluormethoxy steht,
- R⁵: bevorzugt für Chlor, Fluor oder Brom steht,
- R⁵: weiterhin bevorzugt für Iod oder Cyano steht,
zwei benachbarte Reste R⁵ für -(CH=CH-)₂ stehen,
- n: für 1 oder 2 steht,
- n: bevorzugt für 2 steht,
- X: für O steht,
- R⁶: für Methyl oder Chlor steht,
- Q_{X}: für Pyrazol, wie in Anspruch 1 definiert steht, welches durch die Gruppe 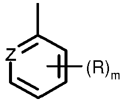
einfach substituiert ist, wobei Z, R und m die angegebenen allgemeinen bzw. die bevorzugten oder besonders bevorzugten Bedeutungen haben können,
- A: für CH₂, CH(CH₃) oder -CH₂O-steht,
- R⁷: für den Rest
steht, 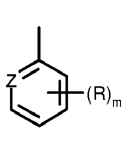
- R: unabhängig voneinander für Fluor, Chlor oder Brom steht,
- R: bevorzugt für Chlor steht,
- m: für 1 steht,
- Z: für N, CCl oder CH steht,

R⁸ für Wasserstoff, für gegebenenfalls einfach oder mehrfach, gleich oder verschieden substituiertes Methyl, Ethyl, iso-Propyl, tert-Butyl steht, wobei die Substituenten unabhängig voneinander ausgewählt sein können aus Halogen, Cyano, Phenyl oder Pyridyl, wobei Phenyl bzw. Pyridyl gegebenenfalls einfach oder mehrfach, gleich oder verschieden substituiert ist durch Wasserstoff, Trifluormethyl, Cyano, Fluor, Chlor, Brom oder Trifluormethoxy,
R⁸ weiterhin für Phenyl, Pyridyl oder für einem 3- bis 6-gliedrigen gesättigten Heterocyclus, enthaltend 1-2 Heteroatome aus der Reihe N,S,O steht, wobei der Phenyl- oder Pyridylring bzw. Heterocyclus gegebenenfalls einfach oder mehrfach, gleich oder verschieden substituiert ist, und wobei die Substituenten unabhängig voneinander ausgewählt sein können aus Wasserstoff, C₁-C₆-Alkyl, C₁-C₆-Haloalkyl, Halogen oder Cyano,
R⁹ für gegebenenfalls einfach oder mehrfach, gleich oder verschieden substituiertes Methyl, Ethyl, iso-Propyl oder tert-Butyl, steht, wobei die Substituenten unabhängig voneinander ausgewählt sein können aus Halogen, Cyano, Phenyl oder Pyridyl, wobei Phenyl bzw. Pyridyl gegebenenfalls einfach oder mehrfach, gleich oder verschieden substituiert ist durch Trifluormethyl, Cyano, Fluor, Chlor oder Trifluormethoxy,
R⁹ weiterhin für Phenyl, Pyridyl oder für einen 3- bis 6-gliedrigen gesättigten Heterocyclus, enthaltend 1-2 Heteroatome aus der Reihe N,S,O, stehen, wobei der Phenyl- oder Pyridylring bzw. der Heterocyclus gegebenenfalls einfach oder mehrfach, gleich oder verschieden substituiert ist, und wobei die Substituenten unabhängig voneinander ausgewählt sein können aus Wasserstoff, C₁-C₆-Alkyl, C₁-C₆-Haloalkyl, Halogen oder Cyano,
Q_{Y} für einen einfach oder mehrfach, gleich oder verschieden substituierten heteroaromatischen Ring der Reihe Q-58, Q-59, Q62, Q63, , wobei die Substituenten unabhängig voneinander ausgewählt sein können aus Methyl, Ethyl, cyclo-Propyl, tert-Butyl, Chlor, Fluor, Iod, Brom, Cyano, Nitro, Difluormethyl, Trifluormethyl, Pentafluorethyl., n-Heptafluorpropyl oder iso-Heptafluorpropyl oder wobei die Substituenten unabhängig voneinander ausgewählt sein können aus Phenyl oder einem 5-oder 6-gliedrigen heteroaromatischen Ring, wobei die Substituenten unabhängig voneinander ausgewählt sein können aus Methyl, Ethyl, cyclo-Propyl, tert-Butyl, Chlor, Fluor, Iod, Brom, Cyano, Nitro, Difluormethyl, Trifluormethyl, Pentafluorethyl., n-Heptafluorpropyl und iso-Heptafluorpropyl,

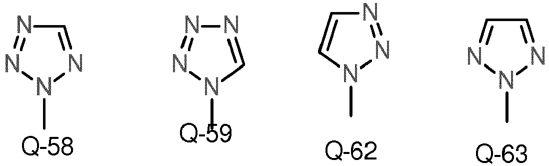

die Verbindungen der allgemeinen Formel (I) außerdem N-Oxide und Salze umfassen.

Die Verbindungen der Formel (I) können gegebenenfalls in verschiedenen polymorphen Formen oder als Mischung verschiedener polymorpher Formen vorliegen. Sowohl die reinen Polymorphe als auch die Polymorphgemische sind Gegenstand der Erfindung und können erfindungsgemäß verwendet werden.

Die Verbindungen der Formel (I) umfassen gegebenenfalls Diastereomere oder Enantiomere.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Insbesondere können die Verbindungen der Formeln (I) in Form verschiedener Regioisomere vorliegen. Beispielsweise in Form von Mischungen aus Verbindungen mit der Definition Q62 bzw. Q63 oder in Form von Mischungen aus Q58 und 59. Erfindungsgemäß umfasst sind daher auch Mischungen aus Verbindungen der Formeln (I), wobei Q_{Y} die Bedeutungen Q62 und Q63, sowie Q58 und Q59 hat und die Verbindungen in verschiedenen Mischungsverhältnissen vorliegen können. Bevorzugt sind dabei Mischungsverhältnisse aus Verbindungen der Formel (I), worin der Rest Q_{Y} für Q62 oder für Q58 steht zu Verbindungen der Formel (I) worin der Rest QY für Q63 oder für Q59 steht, von 60:40 bis 99:1, besonders bevorzugt von 70:30 bis 97:3, ganz besonders bevorzugt von 80:20 bis 95:5. Insbesonders bevorzugt sind die folgenden Mischungsverhältnisse einer Verbindung der Formel (I), wobei Q_{Y} die Bedeutung Q62 oder Q58 hat zur Verbindung der Formel (I), wobei Q_{Y} die Bedeutung Q63 oder Q59 hat: 80:20; 81:19; 82:18; 83:17; 84:16; 85:15, 86:14; 87:13; 88:12; 89:11; 90:10, 91:9; 92:8; 93:7; 96:6; 95:5.

### Herstellverfahren

Die Verbindungen der allgemeinen Formel (I) können erhalten werden, in dem man
(A) Aniline der Formel (II) 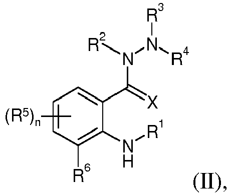 in welcher R¹, R², R³, R⁴, R⁵, R⁶, X und n die oben angegebenen Bedeutungen haben, beispielsweise mit Carbonsäurechloriden der Formel (III) 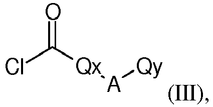 wobei
   Qx, A und Qy die oben angegebenen Bedeutungen haben,
   in Gegenwart eines Säurebindemittels umsetzt; oder
(B) Aniline der Formel (II) 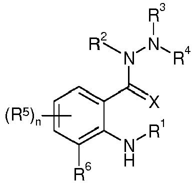 in welcher R¹, R², R³, R⁴, R⁵, R⁶, X und n die oben angegebenen Bedeutungen haben, beispielsweise mit einer Carbonsäure der Formel (IV) 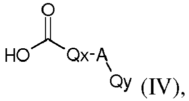 wobei
   Qx, A und Qy die oben angegebenen Bedeutungen haben,
   in Gegenwart eines Kondensationsmittels umsetzt; oder
(C),
   beispielsweise Benzoxazinone der Formel (V) 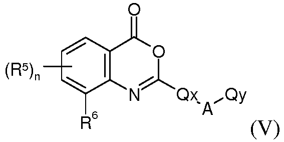 in welcher R⁵, R⁶, Qx, A, Qy und n die oben angegebenen Bedeutungen haben, mit einem Hydrazin der Formel (VI) 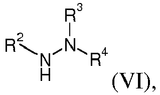 in welcher R², R³, R⁴ die oben angegebenen Bedeutungen haben, in Gegenwart eines Verdünnungsmittels umsetzt; oder
(D) Anthranilsäurehydrazide der Formel (VII) 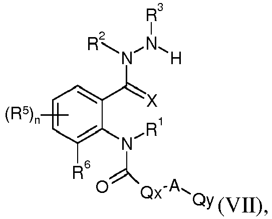 in welcher R¹, R², R³, R⁵, R⁶, X, n, Qx, A, Qy die oben angegebenen Bedeutungen haben,
   mit einem Baustein Y-R⁴ umsetzt, wobei R⁴ die oben angegebene Bedeutung hat und Y eine geeignete Abgangsgruppe wie Halogen oder Alkoxy darstellt;
   oder
(E) Anthranilsäurehydrazide der Formel (VII) 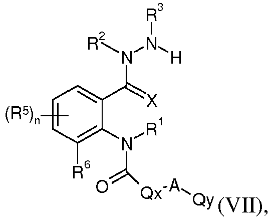 in welcher R¹, R², R³, R⁵, R⁶, X, n, Qx, A, Qy die oben angegebenen Bedeutungen haben,
   mit einem Säureanhydrid der allgemeinen Formel (C(=O)-R⁸)₂O oder (C(=O)-OR⁹)₂O
   oder mit einem Isocyanat der Formel O=C=NR¹¹R¹²,
   wobei R⁸, R⁹, R¹¹, R¹² die oben angegebenen Bedeutungen haben, umsetzt
   zu erfindungsgemäßen Verbindungen der Formel (I).

Insbesondere können Verbindungen der allgemeinen Formel (I), in welchen Qx für 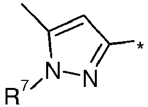 steht, wobei * die Bindung an A kennzeichnet, erhalten werden, in dem man
(A-1) Aniline der Formel (II) 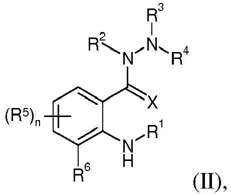 in welcher R¹, R², R³, R⁴, R⁵, R⁶, X und n die oben angegebenen Bedeutungen haben, beispielsweise mit Carbonsäurechloriden der Formel (III-1) 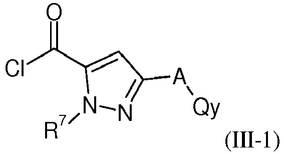 in welcher R⁷, A und Qy die oben angegebenen Bedeutungen haben,
   in Gegenwart eines Säurebindemittels umsetzt; oder
(B-1) Aniline der Formel (II) 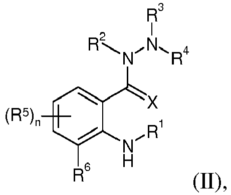 in welcher R¹, R², R³, R⁴, R⁵, R⁶, X und n die oben angegebenen Bedeutungen haben, beispielsweise mit einer Carbonsäure der Formel (IV-1) 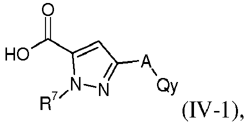 in welcher R⁷, A und Qy die oben angegebenen Bedeutungen haben,
   in Gegenwart eines Kondensationsmittels umsetzt; oder
(C-1) zur Synthese von Anthranilamiden der Formel (I), in welcher R¹ für Wasserstoff steht, Benzoxazinone der Formel (V-1) 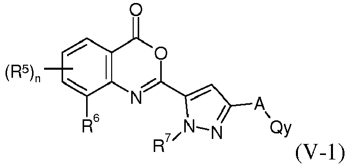 in welcher R⁵, R⁶, R⁷, A, Qy und n die oben angegebenen Bedeutungen haben,
   mit einem Hydrazin der Formel (VI) 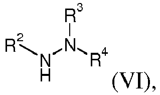 in welcher R², R³, R⁴ die oben angegebenen Bedeutungen haben,
   in Gegenwart eines Verdünnungsmittels umsetzt; oder
(D) Anthranilsäurehydrazide der Formel (VII-1) 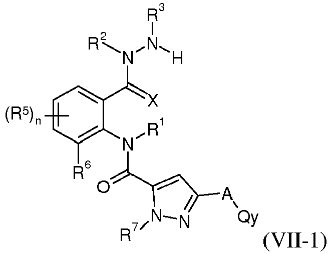 mit einem Baustein Y-R⁴ umsetzt, wobei R⁴ die oben angegebene Bedeutung hat und Y eine geeignete Abgangsgruppe wie Halogen oder Alkoxy darstellt,
   oder
(E) Anthranilsäurehydrazide der Formel (VII-1) 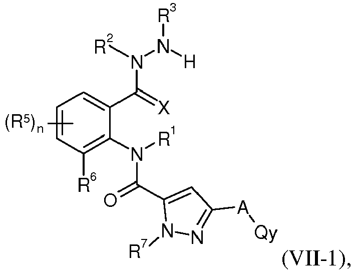 in welcher R¹, R², R³, R⁵, R⁶, X, n, A und Qy die oben angegebenen Bedeutungen haben,
   mit einem Säureanhydrid der allgemeinen Formel (C(=O)-R⁸)₂O oder (C(=O)-OR⁹)₂O
   oder mit einem Isocyanat der Formel O=C=NR¹¹R¹²,
   wobei R⁸, R⁹, R¹¹, R¹² die oben angegebenen Bedeutungen haben, umsetzt
   zu erfindungsgemäßen Verbindungen der Formel (I).

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Anoplura (Phthiraptera) z.B. Damalinia spp., Haematopinus spp., Linognathus spp., Pediculus spp., Trichodectes spp..

Aus der Klasse der Arachnida z.B. Acarus spp., Aceria sheldoni, Aculops spp., Aculus spp., Amblyomma spp., Amphitetranychus viennensis, Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Halotydeus destructor, Hemitarsonemus spp., Hyalomma spp., Ixodes spp., Latrodectus mactans, Metatetranychus spp., Nuphersa spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp..

Aus der Ordnung der Chilopoda z.B. Geophilus spp., Scutigera spp..

Aus der Ordnung der Coleoptera z.B. Acalymma vittatum, Acanthoscelides obtectus, Adoretus spp., Agelastica alni, Agriotes spp., Amphimallon solstitialis, Anobium punctatum, Anoplophora spp., Anthonomus spp., Anthrenus spp., Apion spp., Apogonia spp., Atomaria spp., Attagenus spp., Bruchidius obtectus, Bruchus spp., Cassida spp., Cerotoma trifurcata, Ceutorrhynchus spp., Chaetocnema spp., Cleonus mendicus, Conoderus spp., Cosmopolites spp., Costelytra zealandica, Ctenicera spp., Curculio spp., Cryptorhynchus lapathi, Cylindrocopturus spp., Dermestes spp., Diabrotica spp., Dichocrocis spp., Diloboderus spp., Epilachna spp., Epitrix spp., Faustinus spp., Gibbium psylloides, Hellula undalis, Heteronychus arator, Heteronyx spp., Hylamorpha elegans, Hylotrupes bajulus, Hypera postica, Hypothenemus spp., Lachnosterna consanguinea, Lema spp., Leptinotarsa decemlineata, Leucoptera spp., Lissorhoptrus oryzophilus, Lixus spp., Luperodes spp., Lyctus spp.,

Megascelis spp., Melanotus spp., Meligethes aeneus, Melolontha spp., Migdolus spp., Monochamus spp., Naupactus xanthographus, Niptus hololeucus, Oryctes rhinoceros, Oryzaephilus surinamensis, Oryzaphagus oryzae, Otiorrhynchus spp., Oxycetonia jucunda, Phaedon cochleariae, Phyllophaga spp., Phyllotreta spp., Popillia japonica, Premnotrypes spp., Psylliodes spp., Ptinus spp., Rhizobius ventralis, Rhizopertha dominica, Sitophilus spp., Sphenophorus spp., Sternechus spp., Symphyletes spp., Tanymecus spp., Tenebrio molitor, Tribolium spp., Trogoderma spp., Tychius spp., Xylotrechus spp., Zabrus spp..

Aus der Ordnung der Collembola z.B. Onychiurus armatus.

Aus der Ordnung der Diplopoda z.B. Blaniulus guttulatus.

Aus der Ordnung der Diptera z.B. Aedes spp., Agromyza spp., Anastrepha spp., Anopheles spp., Asphondylia spp., Bactrocera spp., Bibio hortulanus, Calliphora erythrocephala, Ceratitis capitata, Chironomus spp., Chrysomyia spp., Cochliomyia spp., Contarinia spp., Cordylobia anthropophaga, Culex spp., Cuterebra spp., Dacus oleae, Dasyneura spp., Delia spp., Dermatobia hominis, Drosophila spp., Echinocnemus spp., Fannia spp., Gastrophilus spp., Hydrellia spp., Hylemyia spp., Hyppobosca spp., Hypoderma spp., Liriomyza spp.. Lucilia spp., Musca spp., Nezara spp., Oestrus spp., Oscinella frit, Pegomyia spp., Phorbia spp., Prodiplosis spp., Psila rosae, Rhagoletis spp., Stomoxys spp., Tabanus spp., Tannia spp., Tetanops spp., Tipula spp..

Aus der Klasse der Gastropoda z.B. Arion spp., Biomphalaria spp., Bulinus spp., Deroceras spp., Galba spp., Lynmaea spp., Oncomelania spp., Pomacea spp., Succinea spp..

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Collaria spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Monalonion atratum, Nezara spp., Oebalus spp., Pentomidae, Piesma quadrata, Piezodorus spp., Psallus spp., Pseudacysta persea, Rhodnius spp., Sahlbergella singularis, Scaptocoris castanea, Scotinophora spp., Stephanitis nashi, Tibraca spp., Triatoma spp.

Aus der Ordnung der Homoptera z.B. Acyrthosipon spp., Acrogonia spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Drosicha spp., Dysaphis spp., Dysmicoccus spp., Empoasca spp., Eriosoma spp., Erythroneura spp., Euscelis bilobatus, Ferrisia spp., Geococcus coffeae, Hieroglyphus spp., Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva spp., Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes spp., Trioza spp., Typhlocyba spp., Unaspis spp., Viteus vitifolii, Zygina spp..

Aus der Ordnung der Hymenoptera z.B. Athalia spp., Diprion spp., Hoplocampa spp., Lasius spp., Monomorium pharaonis, Vespa spp..

Aus der Ordnung der Isopoda z.B. Armadillidium vulgare, Oniscus asellus, Porcellio scaber.

Aus der Ordnung der Isoptera z.B. Acromyrmex spp., Atta spp., Cornitermes cumulans, Microtermes obesi, Odontotermes spp., Reticulitermes spp,

Aus der Ordnung der Lepidoptera z.B. Acronicta major, Adoxophyes spp., Aedia leucomelas, Agrotis spp., Alabama spp., Amyelois transitella, Anarsia spp., Anticarsia spp., Argyroploce spp., Barathra brassicae, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., Choristoneura spp., Clysia ambiguella, Cnaphalocerus spp., Cnephasia spp., Conopomorpha spp., Conotrachelus spp., Copitarsia spp., Cydia spp., Dalaca noctuides, Diaphania spp., Diatraea saccharalis, Earias spp., Ecdytolopha aurantium, Elasmopalpus lignosellus, Eldana saccharina, Ephestia kuehniella, Epinotia spp., Epiphyas postvittana, Etiella spp., Eulia spp., Eupoecilia ambiguella, Euproctis spp., Euxoa spp., Feltia spp., Galleria mellonella, Gracillaria spp., Grapholitha spp., Hedylepta spp., Helicoverpa spp., Heliothis spp., Hofmannophila pseudospretella, Homoeosoma spp., Homona spp., Hyponomeuta padella, Kakivoria flavofasciata, Laphygma spp., Laspeyresia molesta, Leucinodes orbonalis, Leucoptera spp., Lithocolletis spp., Lithophane antennata, Lobesia spp., Loxagrotis albicosta, Lymantria spp., Lyonetia spp., Malacosoma neustria, Maruca testulalis, Mamestra brassicae, Mocis spp., Mythimna separata, Nymphula spp., Oiketicus spp., Oria spp., Orthaga spp., Ostrinia spp., Oulema oryzae, Panolis flammea, Parnara spp., Pectinophora spp., Perileucoptera spp., Phthorimaea spp., Phyllocnistis citrella, Phyllonorycter spp., Pieris spp., Platynota stultana, Plusia spp., Plutella xylostella, Prays spp., Prodenia spp., Protoparce spp., Pseudaletia spp., Pseudoplusia includens, Pyrausta nubilalis, Rachiplusia nu, Schoenobius spp., Scirpophaga spp., Scotia segetum, Sesamia spp., Sparganothis spp., Spodoptera spp., Stathmopoda spp., Stomopteryx subsecivella, Synanthedon spp., Tecia solanivora, Thermesia gemmatalis, Tinea pellionella, Tineola bisselliella, Tortrix spp., Trichoplusia spp., Tuta absoluta, Virachola spp..

Aus der Ordnung der Orthoptera z.B. Acheta domesticus, Blatta orientalis, Blattella germanica, Dichroplus spp., Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella spp..

Aus der Ordnung der Thysanoptera z.B. Anaphothrips obscurus, Baliothrips biformis, Drepanothris reuteri, Enneothrips flavens, Frankliniella spp., Heliothrips spp., Hercinothrips femoralis, Rhipiphorothrips cruentatus, Scirtothrips spp., Taeniothrips cardamoni, Thrips spp..

Aus der Ordnung der Thysanura z.B. Lepisma saccharina.

Zu den pflanzenparasitären Nematoden gehören z.B. Aphelenchoides spp., Bursaphelenchus spp., Ditylenchus spp., Globodera spp., Heterodera spp., Longidorus spp., Meloidogyne spp., Pratylenchus spp., Radopholus similis, Trichodorus spp., Tylenchulus semipenetrans, Xiphinema spp..

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, Alkyl-Aryl- und/oder POP- POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Die Formulierungen enthalten im Allgemeinen zwischen 0,01 und 98 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenern, Düngemitteln, Semiochemicals vorliegen. Insbesondere können die erfindungsgemäßen Wirkstoffe in Kombination mit weiteren (Hilfs-)Mitteln zur Wirkungssteigerung eingesetzt werden.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Emährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Emährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;
Hautflügler wie Sirex juvencus, Urocerus gigas, Urocerus gigas taignus, Urocerus augur;
Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;
Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp..

Aus der Ordnung der Chilopoda z.B. Geophilus spp..

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus.

Aus der Ordnung der Dermaptera z.B. Forficula auricularia.

Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Pemphigus spp., Phylloera vastatrix, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neo-nicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Erläuterung der Herstellverfahren und Zwischenprodukte

Aniline der Formel (II) sind bekannt (z.B. WO2007/043677, WO2008/126858, WO2008/126933) oder können nach bekannten Methoden hergestellt werden.

Carbonsäurechloride der Formel (III), in welchen Qx für Pyrazol steht, sind bekannt (z.B. WO2007/144100) oder können nach bekannten Methoden hergestellt werden.

Carbonsäuren der Formel (IV), in welchen Qx für Pyrazol steht, sind bekannt (z.B. WO2007/144100) oder können nach bekannten Methoden hergestellt werden.

Benzoxazinone der Formel (V), in welchen Qx für Pyrazol steht, sind bekannt (z.B. WO2007/144100) oder können nach bekannten Methoden hergestellt werden.

Hydrazine der Formel (VI) sind bekannt (z.B. WO2007/043677, WO2008/126858, WO2008/126933) oder können nach bekannten Methoden hergestellt werden.

Anthranilsäurehydrazide der Formel (VII) sind neu. Sie können gemäß Verfahren C hergestellt werden.

### Herstellbeispiele (Synthese von Carbonsäuren der Formel (IV-2))

### Beispiel Nr. A: (IX)

### Synthese von Ethyl-2-(brommethyl)-4-(2-chlorphenyl)pyrimidin-5-carboxylat

820 mg (2,96 mmol) Ethyl-4-(2-chlorphenyl)-2-methylpyrimidin-5-carboxylat und 633 mg (3,55 mmol) N-Brom-succinimid wurden in 18 mL Tetrachlorkohlenstoff gelöst und unter Rühren zum sieden erhitzt. In der Siedehitze wurden portionsweise 49 mg (0,29 mmol) 2,2'-Azobis-2-methylpropannitril über 5 Stunden zugegeben und für eine weitere Stunde refluxiert. Nach Abkühlen auf Raumtemperatur wurde die Reaktionslösung filtriert und die Mutterlauge im Vakuum vom Lösungsmittel befreit. Durch chromatographische Reinigung wurde das gewünschte Produkt isoliert.

(logP: 3,32; MH⁺: 357; ¹H-NMR (400 MHz, DMSO, δ, ppm): 1,00 (t, 3H), 4,12 (q, 2H), 4,80 (s, 2H), 7,50 (m, 4 H), 9,30 (s, 1H).

### Beispiel Nr. B: (X)

### Synthese von Ethyl-4-(2-chlorphenyl)-2-{[5-(trifluormethyl)-2H-tetrazol-2-yl]methyl}pyrimidin-5-carboxylat

345 mg (0,97 mmol) Ethyl-2-(brommethyl)-4-(2-chlorphenyl)pyrimidin-5-carboxylat wurden zu einer Lösung aus 200 mg (1,45 mmol) Trifluormethyltetrazol in 10 mL Acetonitril gegeben. Anschließend wurden 174 mg (1,26 mmol) Kaliumcarbonat zur Reaktionslösung gegeben und für 6 Stunden bei 60 °C gerührt. Nach Abkühlen auf Raumtemperatur wurde die Reaktionslösung filtriert und die Mutterlauge im Vakuum vom Lösungsmittel befreit. Der Rückstand wurde mit 10 mL Wasser versetzt und gegen Essigester (3 x 20 mL) extrahiert. Die vereinigten organischen Phasen wurden über Natriumsulfat getrocknet. Das Lösungsmittel wurde im Vakuum entfernt und der Rückstand chromatographisch aufgereinigt. (logP: 3,56; MH⁺: 413; ¹H-NMR (400 MHz, DMSO, δ, ppm): 0,99 (t, 3H), 4,13 (q, 2H), 6,62 (s, 2H), 7,50 (m, 4 H), 9,28 (s, 1H).

### Beispiel Nr. C: (IV-2)

### Synthese von 4-(2-Chlorphenyl)-2-{[5-(trifluormethyl)-2H-tetrazol-2-yl]methyl}pyrimidin-5-carbonsäure

1,74 g (4,21 mmol) Ethyl-4-(2-chlorphenyl)-2-{[5-(trifluormethyl)-2H-tetrazol-2-yl]methyl}pyrimidin-5-carboxylat wurden in 12 mL Ethanol gelöst und bei 0 °C tropfenweise mit 45%iger Natronlauge (5,05 mmol) versetzt. Die Reaktion wurde eine Stunde nachgerührt und auf RT erwärmt. Anschließend wurde das Ethanol unter vermindertem Druck entfernt und der Rückstand wurde mit Eiswasser (10 mL) versetzt. Die wässrige Phase wurde gegen Essigester extrahiert. Die organische Phase wurde verworfen. Anschließend wurde die wässrige Phase mit Salzsäure auf pH 3 eingestellt und nochmals gegen Essigester (3 x 20 mL) extrahiert. Die vereinigten organischen Phasen wurden über Natriumsulfat getrocknet. (logP: 2,50; MH⁺: 385; ¹H-NMR (400 MHz, DMSO, δ, ppm): 6,59 (s, 2H), 7,48 (m, 4 H), 9,27 (s, 1H).

### Herstellungsbeispiele

Gemäß den oben beschriebenen Herstellverfahren lassen sich die Verbindungen der Formel (I) erhalten, beispielsweise die folgenden Verbindungen der Formel (I):

### Beispiel Nr. 1:

### Synthese von N'-(5-Chloro-2-{[2-(3-chloro-pyridin-2-yl)-5-(5-heptalluoropropyl-tetrazol-2-ylmethyl)-2H-pyrazole-3-carbonyl]-amino}-3-methyl-benzoyl)-N-methyl-hydrazincarbonsäure-methylester

250 mg (0.40 mmol) 6-Chloro-2-[2-(3-chloro-pyridin-2-yl)-5-(5-heptafluoropropyl-tetrazol-2-ylmethyl)-2*H*-pyrazol-3-yl]-8-methyl-3,1-benzoxazin-4-on wurden in 20 ml Tetrahydrofuran gelöst und mit 209 mg (2.06 mmol) *N*-Methyl-hydrazincarbonsäure-methylester versetzt. Es wurde zunächst 3h bei Raumtemperatur gerührt und anschließend 16h unter Rückfluß erhitzt.

Nach dem Abkühlen wurde das Reaktionsgemisch im Vakuum vom Lösungsmittel befreit. Durch chromatographische Reinigung des Rückstands wurde das gewünschte Produkt isoliert (logP: 3.66; MH⁺: 727; ¹H-NMR (400 MHz, DMSO, δ, ppm): 2.07 (s, 3H), 2.88 (s, 3H), 3.45 (br s, 3H), 6.35 (s, 2H), 7.29 (bs, 1H), 7.38 (s, 1H), 7.54 (d, 1H), 7.58 (dd, 1H), 8.13 (dd, 1H), 8.45 (dd, 1H), 10.24 (s, 1H), 10.46 (s, 1H)).

### Beispiel Nr. 2:

### N'-(2-{[2-(3-Chloro-pyridin-2-yl)-5-(5-heptafluoropropyl-tetrazol-2-ylmethyl)-2H-pyrazole-3-carbonyl]-amino}-5-iodo-3-methyl-benzoyl)-hydrazincarbonsäure-methylester

583 mg (0.78 mmol) 2-(3-Chloro-pyridin-2-yl)-5-(5-heptafluoropropyl-tetrazol-2-ylmethyl)-2*H-*pyrazole-3-carbonsäure-(2-hydrazinocarbonyl-4-iodo-6-methyl-phenyl)-amid wurden in 25 ml Pyridin gelöst und tropfenweise mit 84 mg (0.89 mmol) Chlorameisensäuremethylester versetzt. Das Gemisch wurde 2 h bei Raumtemperatur gerührt und im Vakuum vom Lösungsmittel befreit. Durch chromatographische Reinigung des Rückstands wurde das gewünschte Produkt isoliert (logP: 3.58; MH⁺: 805; ¹H-NMR (400 MHz, DMSO, δ, ppm): 2.10 (s, 3H), 3.59 (br s, 3H), 6.34 (s, 2H), 7.34 (s, 1H), 7.58 (dd, 1H), 7.68 (br s, 1H), 7.81 (d, 1H), 8.14 (dd, 1H), 8.47 (dd, 1H), 9.24 (bs, 1H), 10.08 (s, 1H), 10.18 (s, 1H)).

Die folgenden Beispiele und Vergleichsbeispiele können auf analoge Art und Weise erhalten werden: Angegeben sind dabei für das Beispiel Nummer 1 die vollständigen NMR-Signale, für die weiteren Beispiele eine Kombination aus logP-Wert, Masse (MH⁺) und diejenigen NMR-Signale, die sich auf den im Verfahren zuletzt eingeführten Molekülteil beziehen.

| Nr. | Struktur | logP | MH⁺ | ¹H-NMR (400 MHz, DMSO, δ, ppm), ausgewählte Signale |
|---|---|---|---|---|
| 1 | 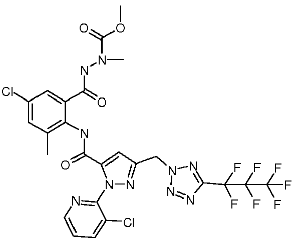 | 3.66 | 727 | 2.07 (s, 3H), 2.88 (s, 3H), 3.45 (br s, 3H), 6.35 (s, 2H), 7.29 (bs, 1H), 7.38 (s, 1H), 7.54 (d, 1H), 7.58 (dd, 1H), 8.13 (dd, 1H), 8.45 (dd, 1H), 10.24 (s, 1H), 10.46 (s, 1H) |
| 2 | 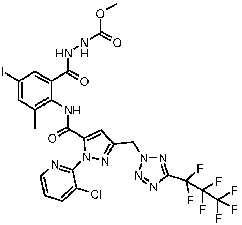 | 3.58 | 805 | 2.10 (s, 3H), 3.59 (br s, 3H), 6.34 (s, 2H), 7.34 (s, 1H), 7.58 (dd, 1H), 7.68 (br s, 1H), 7.81 (d, 1H), 8.14 (dd, 1H), 8.47 (dd, 1H), 9.24 (bs, 1H), 10.08 (s, 1H), 10.18 (s, 1H) |
| 3 | 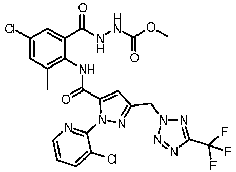 | 2.73 (sauer) | 613 | 3.11 (br s, 3H, OMe), 8.95 (s, NH), 9.95 (s, NH), 10.02 (s, NH) |
| 4 | 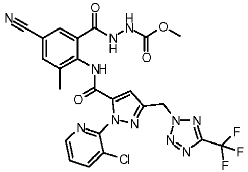 | 2.41 (sauer) | 604 | 3.57 (br s, 3H, OMe), 8.99 (s, NH), 10.08 (s, NH), 10.29 (s, NH) |
| 5 | 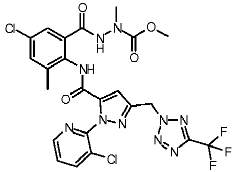 | 2.96 (sauer) | 627 | 2.93 (s, 3H, NMe), 3.52 (br s, 3H, OMe), 10.05 (s, NH). 10.29 (s, NH) |
| 6 | 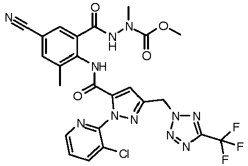 | 2.63 (sauer) | 618 | 2.93 (s, 3H, NMe), 3.59 (br s, 3H, OMe), 10.29 (s, NH), 10.40 (s, NH) |
| 7 | 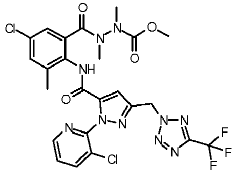 | 3.39 (sauer) | 641 | 2.73; 2.82; 2.92 (s, 3H, NMe), 3.42; 3.62 (br s, 3H, OMe) |
| 8 | 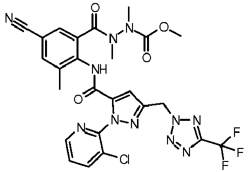 | 2.97 (sauer) | 632 | 2.76; 2.82; 2.91 (s, 3H, NMe), 3.42; 3.61 (br s, 3H, OMe) |
| 9 | 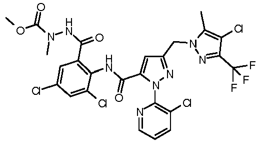 | 4.54 | 853 | 2.85 (s, 3H), 3.43; 3.60 (br s, 3H), 10.31 (s, NH), 10.43 (s, NH), |
| 0 | 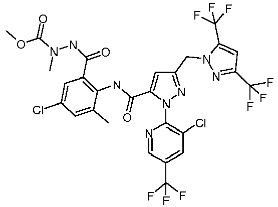 | 4.35 | 761 | 2.86 (s, 3H), 3.44; 3.66 (br s, 3H), 10.35 (s, NH), 10.47 (s, NH) |
| 1 | 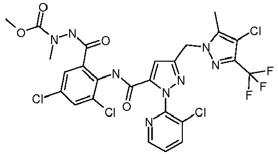 | 3.55 | 693 | 2.86 (s, 3H), 3.44; 3.71 (br s, 3H), 10.48 (s, NH), 10.58 (s, NH) |
| 2 | 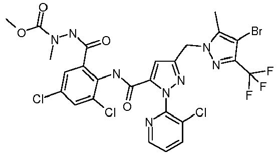 | 3.57 | 737 | 2.87 (s, 3H), 3.45; 3.71 (br s, 3H), 10.49 (s, NH), 10.58 (s, NH) |
| 3 | 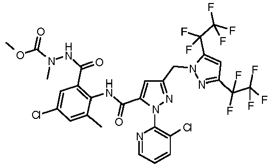 | 4.32 | 793 | 2.86 (s, 3H), 3.45; 3.71 (br s, 3H), 10.24 (s, NH), 10.46 (s, NH) |
| 4 | 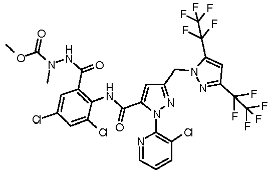 | 4.30 | 815 | 2.86 (s, 3H), 3.45; 3.62 (br s, 3H), 10.51 (s, NH), 10.58 (s, NH) |
| 5 | 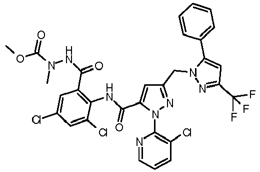 | 3.82 | 721 | 2.86 (s, 3H), 3.45; 3.62 (br s, 3H), 10.51 (s, NH), 10.58 (s, NH) |
| 6 | 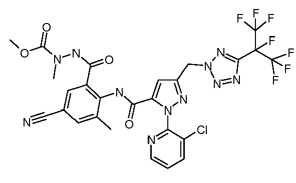 | 3.26 | 718 | 2.87 (s, 3H), 3.46; 3.65 (br s, 3H), 10.49 (s, NH), 10.55 (s, NH) |
| 17 | 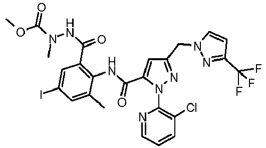 | 3.01 | 717 | 2.87 (s, 3H), 3.44; 3.64 (br s, 3H), 10.16 (s, NH), 10.39 (s, NH) |
| 18 | 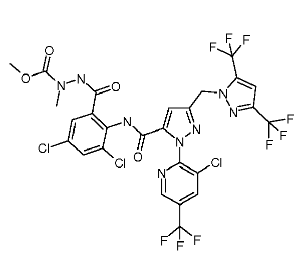 | 4.32 | 781 | CD₃CN: 2.99 (s, 3H), 3.46; 3.66 (br s, 3H), 9.02 (s, NH) |
| 19 | 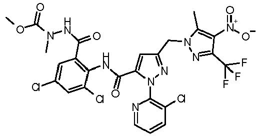 | 3.11 | 706 | 2.86 (s, 3H), 3.47; 3.62 (br s, 3H), 10.47 (s, NH), 10.57 (s, NH) |
| 20 | 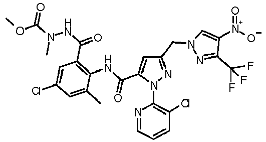 | 3.07 | 670 | CD₃CN: 3.04 (s, 3H), 3.48; 3.66 (br s, 3H), 8.76 (s, NH), 9.06 (s, NH) |
| 21 | 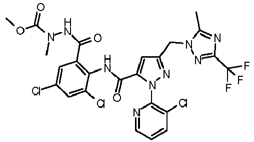 | 2.51 | 662 | 2.87 (s, 3H), 3.45; 3.64 (br s, 3H), 10.47 (s, NH), 10.56 (s, NH) |
| 22 | 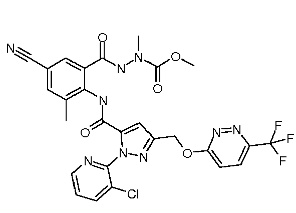 | 2.53 (sauer) | 644 | 2.88 (s, 3H, NMe), 3.50 (br s, 3H, OMe), 10.45 (s, NH), 10.55 (s, NH) |
| 23 | 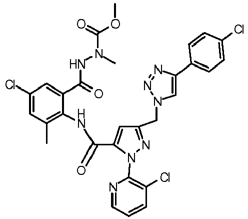 | 3.14 | 668 | 2.87 (s, 3H), 3.58 (s, 3H) |
| 24 | 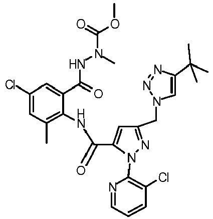 | 2.53 | 614 | 2.87 (s, 3H), 3.58 (s, 3H) |
| 25 | 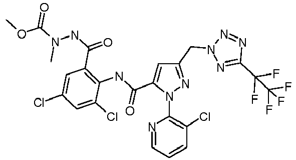 | 3.32 | 697 | 2.87 (s, 3H), 3.45; 3.63 (br s, 3H), 10.54 (s, NH), 10.57 (s, NH) |
| 26 | 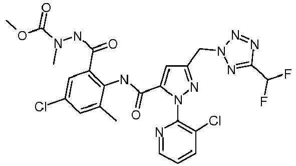 | 2.49 | 609 | 2.88 (s, 3H), 3.47; 3.63 (br s, 3H), 10.22 (s, NH), 10.45 (s, NH) |
| 27 | 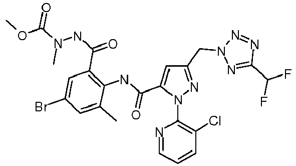 | 2.58 | 653 | 2.88 (s, 3H), 3.48; 3.62 (br s, 3H), 10.21 (s, NH), 10.45 (s, NH) |
| 28 | 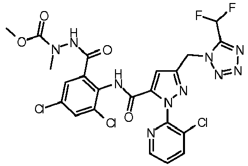 | 2.27 | 629 | 2.87 (s, 3H), 3.47; 3.64 (br s, 3H), 10.52 (s, NH), 10.57 (s, NH) |
| 29 | 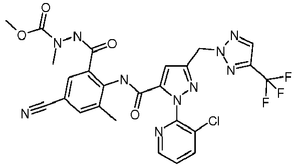 | 2.62 | 617 | 2.87 (s, 3H), 3.46; 3.65 (br s, 3H), 10.43 (s, NH), 10.54 (s, NH) |
| 30 | 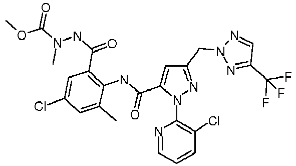 | 2.94 | 626 | 2.88 (s, 3H), 3.44; 3.65 (br s, 3H), 10.19 (s, NH), 10.44 (s, NH) |
| 31 | 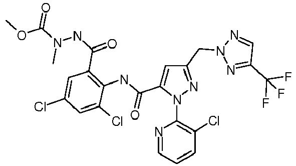 | 2.94 | 648 | 2.87 (s, 3H), 3.44; 3.64 (br s, 3H), 10.50 (s, NH), 10.56 (s, NH) |
| 32 | 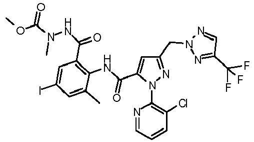 | 2.81 | 718 | 2.87 (s, 3H), 3.45; 3.65 (br s, 3H), 10.16 (s, NH), 10.42 (s, NH) |
| 33 | 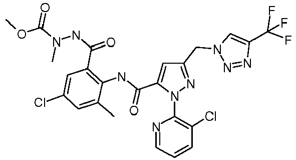 | 2.61 | 626 | CD₃CN: 3.04 (s, 3H), 3.47; 3.65 (br s, 3H), 8.74 (s, NH), 9.03 (s, NH) |
| 34 | 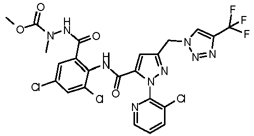 | 2.61 | 646 | CD₃CN: 2.97 (s, 3H), 3.49; 3.65 (br s, 3H), 8.82 (s, NH), 9.03 (s, NH) |
| 35 | 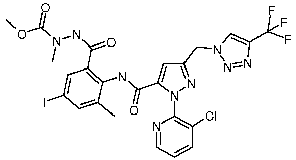 | 3.14 | 718 | 2.87 (s, 3H), 3.44; 3.64 (br s, 3H), 10.16 (s, NH), 10.40 (s, NH) |
| 36 | 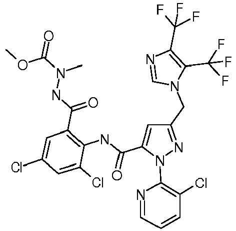 | 3.10 | 713 | 2.86 (s, 3H), 3.47; 3.64 (br s, 3H), 10.47 (s, NH), 10.40 (s, NH) |
| 37 | 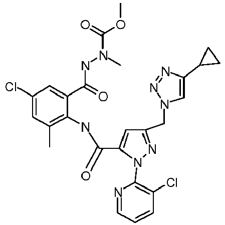 | 2.18 | 598 | 2.88 (s, 3H), 3.61 (s, 3H) |
| 38 | 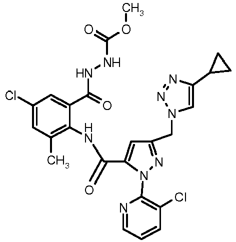 | 2.02 | 584 | 3.61 (s, 3H) |
| 39 | 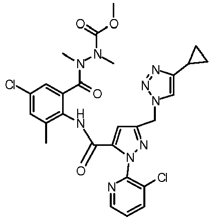 | 2.71 | 612 | 3.60 (s, 3H) |
| 40 | 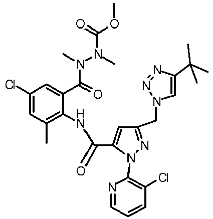 | 2.98 | 628 | 3.59 (s, 3H) |
| 41 | 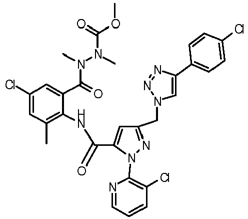 | 3.49 | 682 | 3.60 (s, 3H) |
| 42 | 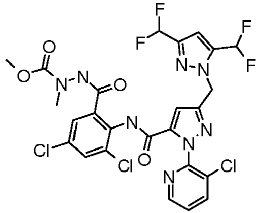 | 2.90 | 677 | 2.86 (s, 3H), 3.43; 3.62 (br s, 3H), 10.49 (s, NH), 10.54 (s, NH) |
| 43 | 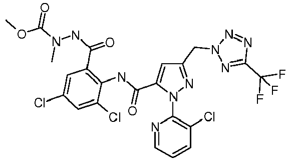 | 2.92 | 647 | 2.87 (s, 3H), 3.46; 3.64 (br s, 3H), 10.53 (s, NH), 10.57 (s, NH) |
| 44 | 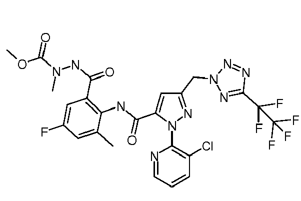 | 3.05 | 661 | 2.91 (s, 3H), 3.44; 3.65 (br s, 3H), 10.17 (s, NH), 10.43 (s, NH) |
| 45 | 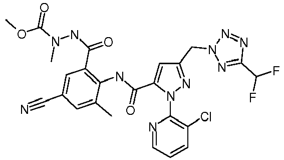 | 2.15 | 600 | 2.87 (s, 3H), 3.47; 3.64 (br s, 3H), 10.46 (s, NH), 10.55 (s, NH) |
| 46 | 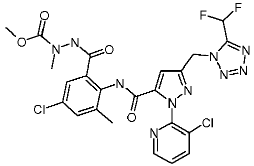 | 2.25 | 609 | 2.88 (s, 3H), 3.40; 3.64 (br s, 3H), 10.21 (s, NH), 10.45 (s, NH) |
| 47 | 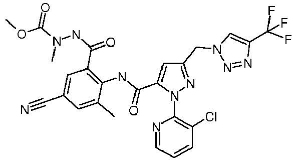 | 2.30 | 617 | CD₃CN: 3.03 (s, 3H), 3.50; 3.65 (br s, 3H), 8.88 (s, NH), 9.35 (s, NH) |
| 48 | 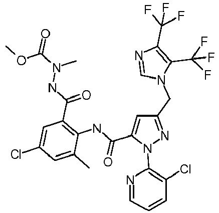 | 3.08 | 693 | 2.86 (s, 3H), 3.47; 3.65 (br s, 3H), 10.18 (s, NH), 10.45 (s, NH) |
| 49 | 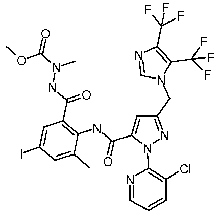 | 3.26 | 785 | 2.86 (s, 3H), 3.47; 3.65 (br s, 3H), 10.15 (s, NH), 10.42 (s, NH) |
| 50 | 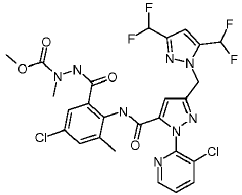 | 2.91 | 659 | 2.88 (s, 3H), 3.44; 3.64 (br s, 3H), 10.19 (s, NH), 10.43 (s, NH) |
| 51 | 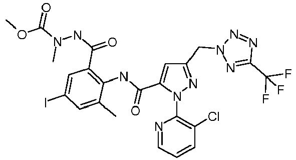 | 3.15 | 719 | 2.87 (s, 3H), 3.45; 3.64 (br s, 3H), 10.20 (s, NH), 10.43 (s, NH) |
| 52 | 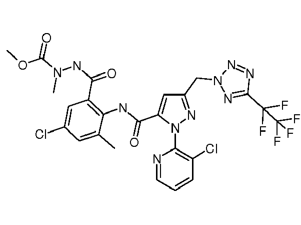 | 3.32 | 677 | 2.88 (s, 3H), 3.45; 3.65 (br s, 3H), 10.24 (s, NH), 10.46 (s, NH) |
| 53 | 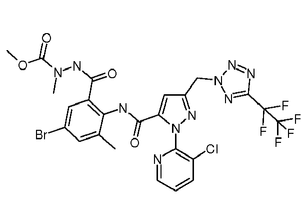 | 3.40 | 721 | 2.88 (s, 3H), 3.45; 3.64 (br s, 3H), 10.23 (s, NH), 10.46 (s, NH) |
| 54 | 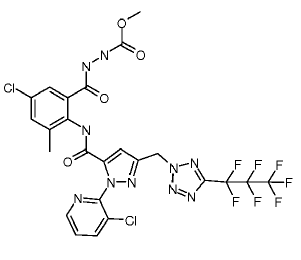 | 3.41 | 713 | 3.61 (bs, 3H) |
| 55 | 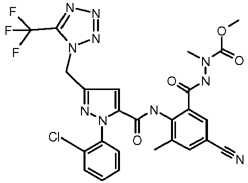 | 2.69 | 617 | 3.49 (br, 3H OCH3); 2.76 (s, 3H CH3); 7,95 (s, NH); 7.77 (s, NH) |
| 56 | 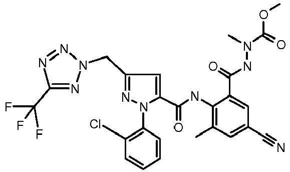 | 2.97 | 617 | 3.57 (br, 3H OCH3); 2.84 (s, 3H CH3); 7,67 - 7.77 (d, NH); 7.79 - 7.95 (d, NH) |
| 57 | 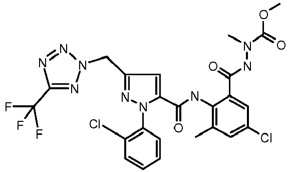 | 3.34 | 627 | 3.34 (br, 3H OCH3); 2.9 (s, 3H CH3); 7.49 - 7.5 (d, NH); 7,54 - 7.57 (d, NH) |
| 58 | 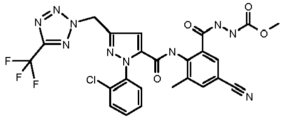 | 2.79 | 604 | 3.52 (br, 3H OCH3); 7.54 - 7.56 (d, NH); 7.75 (s, NH); 7.94 (s, NH) |
| 59 | 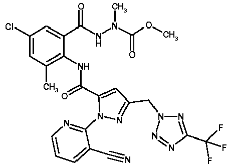 | 2,73 | 618 | 10,57 (s, NH), 10,42 (s, NH), 3,64 und 3,46 (br s, 3H), 2,90 (s, 3H) |
| 60 | 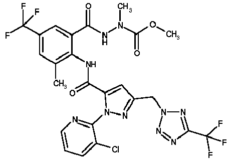 | 3,17 | 661 | |
| 61 | 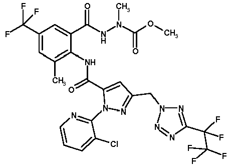 | 3,55 | 711 | 10,57 (s, NH), 10,44 (s, NH), 3,64 und 3,46 (br s, 3H), 2,90 (s, 3H) |
| 62 | 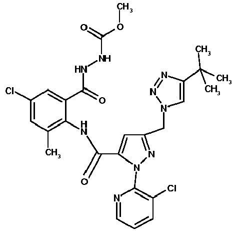 | 2,38 (sauer) | 602 | 1,3 (s, 9H, tBu), 3,6 (br, s, 3H, OMe), 9,3 (br, s, 1H, NH), 11,1 (s, br, 1H, NH) |
| 63 | 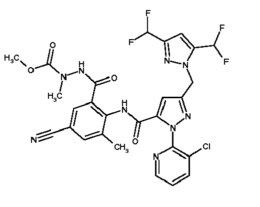 | 2.59 | 648 | 10.52 (s, NH) 10.42 (s, NH) 3.64 und 3.46 (br, s, 3H) 2.87 (s, 3H) |
| 64 | 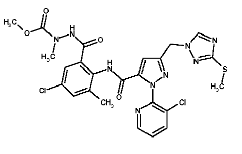 | 2.09 | 604/606 | NMR in CH₃CN: 9.07 (br, NH), 8.83 (s, NH), 3.70 und 3.46 (br, 3H), 3.03 (s, 3H) |
| 65 | 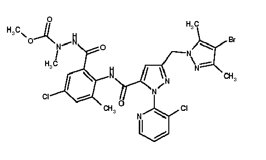 | 2.97 | 665 | 10.43 (s, NH) 10.12 (s, NH) 3.65 und 3.45 (br, s, 3H) 2.88 (s, 3H) |
| 66 | 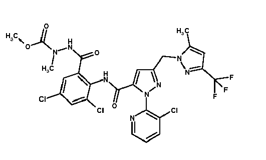 | 3.10 | 659 | 10.54 (s, NH) 10.46 (s, NH) 3.63 und 3.44 (br, s, 3H) 2.86 (s, 3H) |
| 67 | 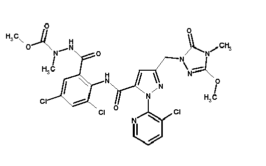 | 1.90 | 638 | 10.56 (s, NH) 10.43 (s, NH) 3.62 und 3.47 (br, s, 3H) 2.87 (s, 3H) |
| 68 | 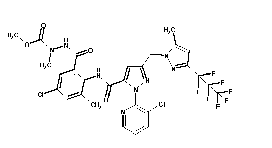 | 3.82 | 739 | NMR in CH₃CN: 8.90 (br, NH), 8.70 (s, NH), 3.70 und 3.45 (br, 3H), 3.03 (s, 3H) |
| 69 | 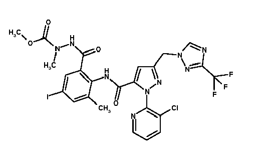 | 2.71 | 718 | NMR in CH₃CN: 9.04 (br, NH), 8.73 (s, NH), 3.69 und 3.50 (br, 3H), 3.04 (s, 3H) |
| 70 | 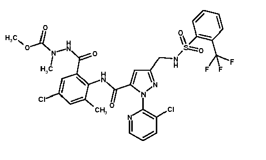 | 2.81 | 714 | NMR in CH₃CN: 9.01 (br, NH), 8.78 (s, NH), 3.65 und 3.52 (br, 3H), 3.06 (s, 3H) |
| 71 | 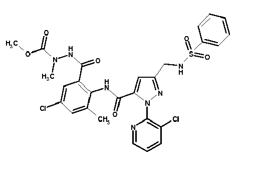 | 2.42 | 646 | 10.45 (s, NH) 10.15 (s, NH) 3.60 und 3.51 (br, s, 3H) 2.90 (s, 3H) |
| 72 | 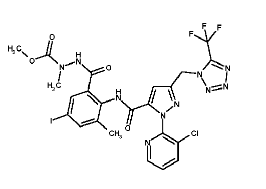 | 2.78 | 719 | 10.43 (s, NH) 10.19 (s, NH) 3.60 und 3.50 (br, s, 3H) 2.87 (s, 3H) |
| 73 | 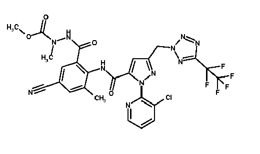 | 2.97 | 668 | 10.55 (s, NH) 10.48 (s, NH) 3.60 und 3.47 (br, s, 3H) 2.87 (s, 3H) |
| 74 | 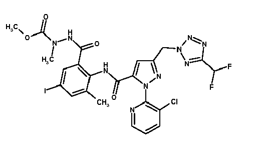 | 2.64 | 701 | 10.41 (s, NH) 10.19 (s, NH) 3.64 und 3.45 (br, s, 3H) 2.87 (s, 3H) |
| 75 | 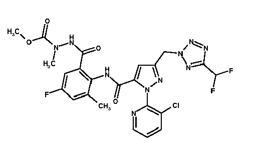 | 2.19 | 593 | 10.41 (s, NH) 10.15 (s, NH) 3.66 und 3.48 (br, s, 3H) 2.90 (s, 3H) |
| 76 | 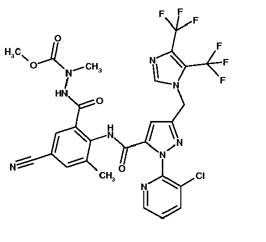 | 2.77 | 684 | 10.55 (s, NH) 10.42 (s, NH) 3.60 und 3.47 (br, s, 3H) 2.87 (s, 3H) |
| 77 | 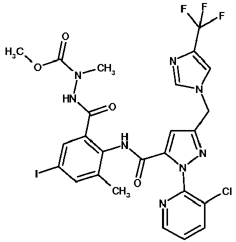 | 2.56 | 717 | 10.45 (s, NH) 10.20 (s, NH) 3.64 und 3.45 (br, s, 3H) 2.86 (s, 3H) |
| 78 | 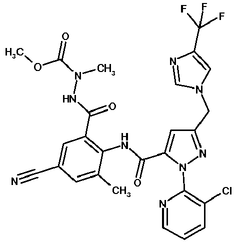 | 2.10 | 616 | NMR in CH₃CN: |
| | | | | 9.40 (br, NH), 8.95 (s, NH), 3.65 und 3.50 (br, 3H), 3.01 (s, 3H) |
| 79 | 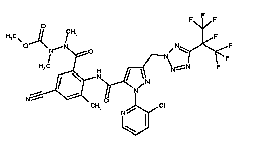 | 3.66 | 732 | 10.62 (s, NH) 10.35 (s, NH) 3.68 und 3.58 (br, s, 3H) 3.06 (s, 3H) |
| 80 | 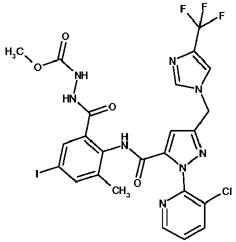 | 2.38 | 703 | NMR in DMF-d₉: 10.34 (s, NH), 10.25 (s, NH), 9.33 (s, NH), 3.51 (s, 3H), 2.95 (s, 3H) |
| 81 | 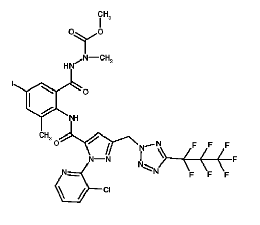 | 3,85 | | |
| 82 | 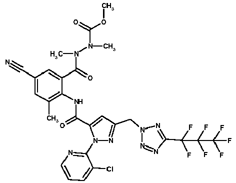 | 3,67 | | |
| 83 | 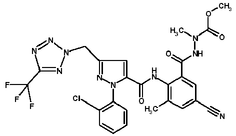 | 2,97 | 620 | 10,37 (br s, NH), 7,95 (br s, NH), 3,30 (br s, 3H), 2,84 (s, 3H) |
| 84 | 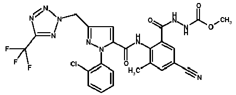 | 2,79 | 603 | 10,22 (br s, NH), 7,94 (br, NH), 3,60 (br, 3H) |
| 85 | 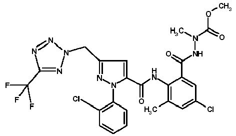 | 3,34 | 627 | 10,13 (br, NH), 7,5 (br, NH), 3,39 (br, 3H), 2,79 (s, 3H) |
| 86 | 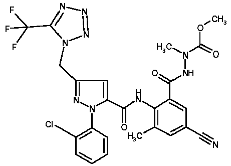 | 2,69 | 620 | 10,37 (br, NH), 7,94 (br, NH), 3,30 (br, 3H), 2,67 (br, 3H), |

### Anwendungsbeispiele und Vergleichsbeispiele

### Beispiel 1 :

### Myzus-Test (MYZUPE Spritzbehandlung)

Lösungsmittel: 78,0 Gewichtsteile Aceton
   1,5 Gewichtsteile Dimethylformamid
Emulgator: 0,5 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Chinakohlblattscheiben (*Brassica pekinensis*), die von allen Stadien der Grünen Pfirsichblattlaus (*Myzus persicae)* befallen sind, werden mit einer Wirkstoffzubereitung der gewünschten Konzentration gespritzt.

Nach 6 Tagen wird die Wirkung in % bestimmt. Dabei bedeutet 100 %, dass alle Blattläuse abgetötet wurden; 0 % bedeutet, dass keine Blattläuse abgetötet wurden.
Bei diesem Test zeigen z. B. die folgenden Verbindungen der Herstellungsbeispiele Wirkung von 80 % bei einer Aufwandmenge von 100 g / ha : Bsp Nr : 39, 65, 77, 81
Bei diesem Test zeigen z. B. die folgenden Verbindungen der Herstellungsbeispiele Wirkung von 90 % bei einer Aufwandmenge von 100 g / ha : Bsp Nr : 11, 20, 21, 32, 47, 54, 61, 78
Bei diesem Test zeigen z. B. die folgenden Verbindungen der Herstellungsbeispiele Wirkung von 100 % bei einer Aufwandmenge von 100 g / ha : Bsp Nr :
   1,3,4,5,6,7,8,12,16,17,22,23,25,26,27,28,29,30,31,33,34,35,36,37,38,41,42,45,46,48,49,50,51,52,53 60, 63,66, 68, 69, 72, 73, 74, 75, 79, 82, 83, 84, 85, 86

### Beispiel 2:

### Phaedon-Test (PHAECO Spritzbehandlung)

Lösungsmittel: 78,0 Gewichtsteile Aceton
   1,5 Gewichtsteile Dimethylformamid
Emulgator: 0,5 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Chinakohlblattscheiben (*Brassica pekinensis*) werden mit einer Wirkstoffzubereitung der gewünschten Konzentration gespritzt und nach dem Abtrocknen mit Larven des Meerrettichblattkäfers (*Phaedon cochleariae*) besetzt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele Wirkung von 100 % bei einer Aufwandmenge von 100 g / ha :
Bsp Nr: 1,2,3,4,5,6,7,8,11,12,13,14,16,17,19,20,22,23,25,26,27,28,29,30,31,32,33,34,35,36,37,38,41, 42,45,46,47,48,49,50,51,52,53,54,59, 60, 61, 63, 66, 68, 69,72, 73, 74, 75, 79, 81, 82, 83, 84, 85, 86

### Beispiel 3:

Spodoptera frugiperda-Test (SPODFR Spritzbehandlung)
Lösungsmittel: 78,0 Gewichtsteile Aceton
   1,5 Gewichtsteile Dimethylformamid
Emulgator: 0,5 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.

Nach 7 Tagen wird die Wirkung in % bestimmt. Dabei bedeutet 100 %, dass alle Raupen abgetötet wurden; 0 % bedeutet, dass keine Raupe abgetötet wurde.
Bei diesem Test zeigt z. B. die folgende Verbindung der Herstellungsbeispiele Wirkung von 83 % bei einer Aufwandmenge von 100 g / ha : Bsp Nr : 77
Bei diesem Test zeigen z. B. die folgenden Verbindungen der Herstellungsbeispiele Wirkung von 100 % bei einer Aufwandmenge von 100 g / ha :
   Bsp Nr : 1,2,3,4,5,6,7,8,11,12,13,14,15,16,17,19,20,21,23,25,26,27,28,29,30,31,32,33,34,35,36,37,38, 41,42,45,46,47,48,49,50,51,52,53,54,59, 60, 61, 62, 63, 65, 66, 68, 69, 72, 73, 74, 75, 79, 81, 82, 83, 84, 85, 86

### Beispiel 4:

### Tetranychus - test, OP-resistent (TETRUR Spritzbehandlung)

Lösungsmittel: 78,0 Gewichtsteile Aceton
   1,5 Gewichtsteile Dimethylformamid
Emulgator : 0,5 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Bohnenblattscheiben *(Phaseolus vulgaris*), die von allen Stadien der Gemeinen Spinnmilbe (*Tetranychus urticae*) befallen sind, werden mit einer Wirkstoffzubereitung der gewünschten Konzentration gespritzt. Nach 6 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigt z. B. die folgende Verbindung der Herstellungsbeispiele eine Wirkung von 90 % bei einer Aufwandmenge von 100 g/ha: 83.

Bei diesem Test zeigt z. B. die folgende Verbindung der Herstellungsbeispiele eine Wirkung von 100 % bei einer Aufwandmenge von 100 g/ha: 59.

Bei diesem Test zeigt z. B. die folgende Verbindung der Herstellungsbeispiele eine Wirkung von 100 % bei einer Aufwandmenge von 500 g/ha: 67.

### Analytische Methoden

Die Bestimmung der in der voranstehenden Tabelle und den Herstellungsbeispielen angegebenen logP Werten erfolgte gemäß EEC Directive 79/831 Annex V.A8 durch HPLC (High Performance Liquid Chromatography) an reversed-phase Säulen (C 18), mit nachfolgenden Methoden:
^{[a]} Die Bestimmung erfolgt im sauren Bereich bei pH 2.3 mit 0,1% wässriger Phosphorsäure und Acetonitril als Eluenten; linearer Gradient von 10% Acetonitril bis 95% Acetonitril.
^{[b]} Die Bestimmung mit der LC-MS im sauren Bereich erfolgt bei pH 2,7 mit 0,1 % wässriger Ameisensäure und Acetonitril (enthält 0,1% Ameisensäure) als Eluenten; linearer Gradient von 10% Acetonitril bis 95% Acetonitril.

Die Kalibrierung erfolgte mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen).

Die lambda-maX Werte wurden an Hand der UV-Spektren von 200 nm bis 400 nm in den Maxima der chromatographischen Signale ermittelt.

Die MH⁺-Signale wurden mit einem Agilent MSD-System mit ESI und positiver oder negativer Ionisation bestimmt.

Die NMR-Spektren wurden
a) mit einem Bruker Avance 400, ausgestattet mit einem Durchflussprobenkopf (60 µl Volumen), bestimmt. Als Lösungsmittel wurden CD₃CN oder d₆-DMSO verwendet, wobei als Referenz Tetramethylsilan (0.00 ppm) eingesetzt wurde.
b) mit einem Bruker Avance II 600 bestimmt. Als Lösungsmittel wurden CD₃CN oder d₆-DMSO verwendet, wobei als Referenz Tetramethylsilan (0.00 ppm) eingesetzt wurde.

Die Aufspaltung der Signale wurde wie folgt beschrieben: s (Singulett), d (Duplett), t (Triplett), q (Quartett), quin (Quintett), m (Multiplett).

## Patentansprüche

1. Anthranilsäurederivate der allgemeinen Formel (I) 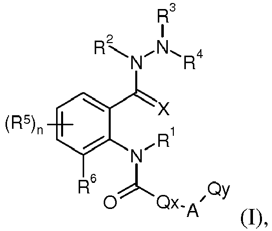 in welcher
R¹ für Wasserstoff steht,
R², R³ unabhängig voneinander für Wasserstoff, Methyl, Ethyl, iso-Propyl, tert-Butyl stehen,
R⁴ für -C(=O)-R⁸, -C(=O)-OR⁹ steht,
R⁵ für Wasserstoff, Methyl, Trifluormethyl, Cyano, Fluor, Chlor, Brom, Iod oder Trifluormethoxy steht,
zwei benachbarte Reste R⁵ ebenfalls für -(CH=CH-)₂ stehen,
n für 2 steht,
X für O steht,
R⁶ für Methyl oder Chlor steht,
Qx für 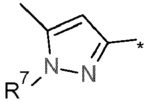
steht, welches durch die Gruppe R⁷ 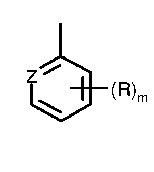 einfach substituiert ist, und wobei * die Bindung an A kennzeichnet,
A für für CH₂, CH(CH₃) oder -CH₂O-steht,
R unabhängig voneinander für Fluor, Chlor oder Brom steht,
m für 1 steht,
Z für N, CCl oder CH steht,
Q_{Y} für einen einfach oder mehrfach, gleich oder verschieden substituierten heteroaromatischen Ring der Reihe, Q-58, Q-59, Q62, Q63 steht, 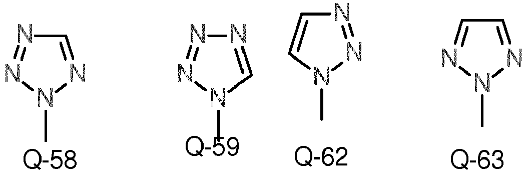 wobei die Substituenten unabhängig voneinander ausgewählt sein können aus Methyl, Ethyl, cyclo-Propyl, tert-Butyl, Chlor, Fluor, Iod, Brom, Cyano, Nitro, Difluormethyl, Trifluormethyl, Pentafluorethyl, n-Heptafluorpropyl oder iso-Heptafluorpropyl
oder wobei die Substituenten unabhängig voneinander ausgewählt sein können aus Phenyl oder einem 5- oder 6-gliedrigen heteroaromatischen Ring, wobei die Substituenten unabhängig voneinander ausgewählt sein können aus Methyl, Ethyl, cyclo-Propyl, tert-Butyl, Chlor, Fluor, Iod, Brom, Cyano, Nitro, Difluormethyl, Trifluormethyl, Pentafluorethyl, n-Heptafluorpropyl und iso-Heptafluorpropy,
R⁸ für Wasserstoff, für gegebenenfalls einfach oder mehrfach, gleich oder verschieden substituiertes Methyl, Ethyl, iso-Propyl, tert-Butyl steht, wobei die Substituenten unabhängig voneinander ausgewählt sein können aus Halogen, Cyano, Phenyl oder Pyridyl, wobei Phenyl bzw. Pyridyl gegebenenfalls einfach oder mehrfach, gleich oder verschieden substituiert ist durch Wasserstoff, Trifluormethyl, Cyano, Fluor, Chlor, Brom oder Trifluormethoxy,
R⁸ weiterhin für Phenyl, Pyridyl oder für einem 3- bis 6-gliedrigen gesättigten Heterocyclus, enthaltend 1-2 Heteroatome aus der Reihe N,S,O steht, wobei der Phenyl- oder Pyridylring bzw. Heterocyclus gegebenenfalls einfach oder mehrfach, gleich oder verschieden substituiert ist, und wobei die Substituenten unabhängig voneinander ausgewählt sein können aus Wasserstoff, C₁-C₆-Alkyl, C₁-C₆-Haloalkyl, Halogen oder Cyano,
R⁹ für gegebenenfalls einfach oder mehrfach, gleich oder verschieden substituiertes Methyl, Ethyl, iso-Propyl oder tert-Butyl, stehen, wobei die Substituenten unabhängig voneinander ausgewählt sein können aus Halogen, Cyano, Phenyl oder Pyridyl, wobei Phenyl bzw. Pyridyl gegebenenfalls einfach oder mehrfach, gleich oder verschieden substituiert ist durch Trifluormethyl, Cyano, Fluor, Chlor oder Trifluormethoxy,
R⁹ weiterhin für Phenyl, Pyridyl oder für einen 3- bis 6-gliedrigen gesättigten Heterocyclus, enthaltend 1-2 Heteroatome aus der Reihe N,S,O, stehen, wobei der Phenyl- oder Pyridylring bzw. der Heterocyclus gegebenenfalls einfach oder mehrfach, gleich oder verschieden substituiert ist, und wobei die Substituenten unabhängig voneinander ausgewählt sein können aus Wasserstoff, C₁-C₆-Alkyl, C₁-C₆-Haloalkyl, Halogen oder Cyano.

2. Verfahren zur Herstellung von Verbindungen der allgemeinen Formel (I) gemäß Anspruch 1, **dadurch gekennzeichnet, dass** man
(A) Aniline der Formel (II) 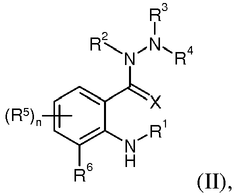 in welcher R¹, R², R³, R⁴, R⁵, R⁶, X und n die Bedeutungen gemäß Anspruch 1 aufweisen, mit Carbonsäurechloriden der Formel (III) 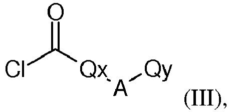 wobei
Qx, A und Qy die Bedeutungen gemäß Anspruch 1 aufweisen,
in Gegenwart eines Säurebindemittels umsetzt; oder
(B) Aniline der Formel (II) 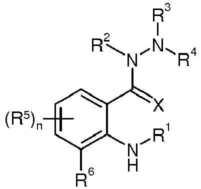 in welcher R¹, R², R³, R⁴, R⁵, R⁶, X und n die Bedeutungen gemäß Anspruch 1 aufweisen, mit einer Carbonsäure der Formel (IV) 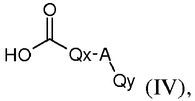 wobei
Qx, A und Qy die Bedeutungen gemäß Anspruch 1 aufweisen,
in Gegenwart eines Kondensationsmittels umsetzt; oder
(C) Benzoxazinone der Formel (V) 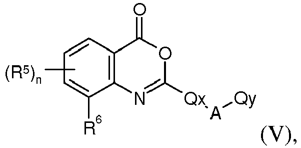 in welcher R⁵, R⁶, Qx, A, Qy und n die Bedeutungen gemäß Anspruch 1 aufweisen,
mit einem Hydrazin der Formel (VI) 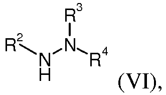 in welcher R², R³, R⁴ die Bedeutungen gemäß Anspruch 1 aufweisen,
in Gegenwart eines Verdünnungsmittels umsetzt; oder
(D) Anthranilsäurehydrazide der Formel (VII) 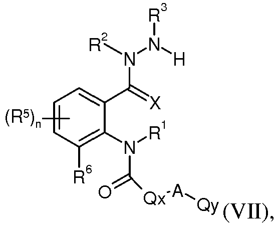 in welcher R¹, R¹, R³, R⁵, R⁶, X, n, Qx, A, Qy die Bedeutungen gemäß Anspruch 1 aufweisen,
mit einem Baustein Y-R⁴ umsetzt, wobei R⁴ die Bedeutung gemäß Anspruch 1 aufweist und Y eine geeignete Abgangsgruppe wie Halogen oder Alkoxy darstellt; oder
(E) Anthranilsäurehydrazide der Formel (VII) 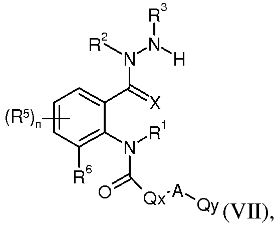 in welcher R¹, R², R³, R⁵, R⁶, X, n, Qx, A, Qy die Bedeutungen gemäß Anspruch 1 aufweisen,
mit einem Säureanhydrid der allgemeinen Formel (C(=O)-R⁸)₂O oder (C(=O)-OR⁹)₂O
wobei R⁸, R⁹ die Bedeutungen gemäß Anspruch 1 aufweisen,
umsetzt zu erfindungsgemäßen Verbindungen der Formel (I).

3. Mischungen von Verbindungen der allgemeinen Formel (I) gemäß Anspruch 1, in welchen Qy für Q62 und Q63 steht, wobei das Verhältnis einer Verbindung der Formel (I), in welchen Qy für Q62 steht, zu einer Verbindung der Formel (I), in welchen Qy für Q63 steht, 60:40 bis 99:1 beträgt.

4. Mischungen von Verbindungen der allgemeinen Formel (I) gemäß Anspruch 1, in welchen Qy für Q58 und Q59 steht, wobei das Verhältnis einer Verbindung der Formel (I), in welchen Qy für Q58 steht, zu einer Verbindung der Formel (I), in welchen Qy für Q59 steht, 60:40 bis 99:1 beträgt.

5. Verbindungen der allgemeinen Formel (VII), 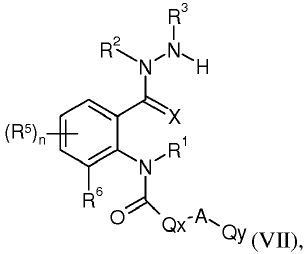 in welcher R¹, R², R³, R⁵, R⁶, X, n, Qx, A, Qy die Bedeutungen gemäß Anspruch 1 aufweisen.

6. Agrochemische Zusammensetzungen enthaltend mindestens eine Verbindung der Formel (I) oder eine Mischung von Verbindungen der Formel (I) gemäß einem der Ansprüche 1, 3 oder 4, sowie Streckmittel und/oder oberflächenaktive Stoffe.

7. Verfahren zur Herstellung agrochemischer Zusammensetzungen, **dadurch gekennzeichnet, dass** mindestens eine Verbindung der allgemeinen Formel (I) oder eine Mischung von Verbindungen der allgemeinen Formel (I) gemäß einem der Ansprüche 1, 3 oder 4, mit Streckmitteln und/oder oberflächenaktiven Stoffen gemischt wird.

8. Verwendung einer Verbindung der allgemeinen Formel (I) gemäß Anspruch 1, einer Mischung von Verbindungen gemäß Anspruch 3 oder 4 oder einer Zusammensetzung gemäß Anspruch 6 zur Bekämpfung tierischer Schädlinge, ausgenommen zur therapeutischen Behandlung des tierischen oder menschlichen Körpers

9. Verfahren zur Bekämpfung tierischer Schädlinge, -ausgenommen zur therapeutischen Behandlung des tierischen oder menschlichen Körpers-**dadurch gekennzeichnet, dass** man eine Verbindung der allgemeinen Formel (I) gemäß Anspruch 1, eine Mischung von Verbindungen der allgemeinen Formel (I) gemäß Anspruch 3 oder 4 oder eine Zusammensetzung gemäß Anspruch 6 auf tierische Schädlinge und/oder phytopathogene Pilze und/oder deren Lebensraum und/oder Saatgut einwirken lässt.

## Claims

1. Anthranilic acid derivatives of the general formula (I) 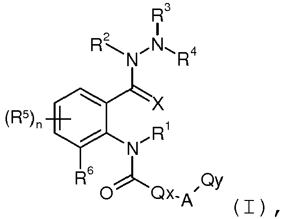 in which
R¹ is hydrogen,
R² and R³ independently of one another are hydrogen, methyl, ethyl, isopropyl, tert-butyl,
R⁴ is -C(=O)-R⁸ or -C(=O)-OR⁹,
R⁵ is hydrogen, methyl, trifluoromethyl, cyano, fluoro, chloro, bromo, iodo or trifluoromethoxy,
two adjacent radicals R⁵ likewise are -(CH=CH-)₂,
n is 2,
X is O,
R⁶ is methyl or chloro,
Qx is 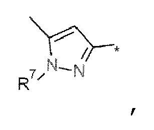
which is singly substituted by the group R⁷ 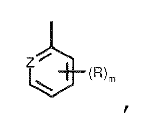 and where * denotes the bond to A,
A is CH₂, CH(CH₃) or -CH₂O-,
R independently at each occurence is fluoro, chloro or bromo,
m is 1,
Z is N, CCl or CH,
Q_{Y} is a singly or multiply identically or differently substituted heteroaromatic ring from the series Q-58, Q-59, Q62, Q63, 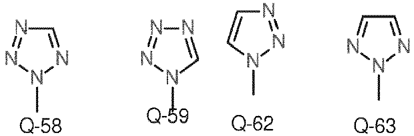 the substituents being selectable independently of one another from methyl, ethyl, cyclopropyl, tert-butyl, chloro, fluoro, iodo, bromo, cyano, nitro, difluoromethyl, trifluoromethyl, pentafluoroethyl, n-heptafluoropropyl or isoheptafluoropropyl,
or the substituents being selectable independently of one another from phenyl or a 5- or 6-membered heteroaromatic ring, where the substituents may be selected independently of one another from methyl, ethyl, cyclopropyl, tert-butyl, chloro, fluoro, iodo, bromo, cyano, nitro, difluoromethyl, trifluoromethyl, pentafluoroethyl, n-heptafluoropropyl and isoheptafluoropropyl,
R⁸ is hydrogen, is methyl, ethyl, isopropyl or tert-butyl optionally singly or multiply identically or differently substituted, the substituents being selectable independently of one another from halogen, cyano, phenyl or pyridyl, where phenyl or pyridyl is optionally substituted one or more times by identical or different hydrogen, trifluoromethyl, cyano, fluoro, chloro, bromo or trifluoromethoxy substituents,
R⁸ additionally is phenyl, pyridyl or is a 3- to 6-membered saturated heterocycle, containing 1-2 heteroatoms from the series N, S and O, the phenyl or pyridyl ring or heterocycle being optionally substituted one or more times by identical or different substituents, and the substituents being selectable independently of one another from hydrogen, C₁-C₆-alkyl, C₁-C₆-haloalkyl, halogen or cyano,
R⁹ are optionally singly or multiply identically or differently substituted methyl, ethyl, isopropyl or tert-butyl, the substituents being selectable independently of one another from halogen, cyano, phenyl or pyridyl, where phenyl or pyridyl is optionally substituted by one or more identical or different trifluoromethyl, cyano, fluoro, chloro or trifluoromethoxy substituents,
R⁹ additionally are phenyl, pyridyl or are a 3- to 6-membered saturated heterocycle containing 1-2 heteroatoms from the series N, S and O, the phenyl or pyridinyl ring or the heterocycle being optionally substituted one or more times by identical or different substituents, and the substituents being selectable independently of one another from hydrogen, C₁-C₆-alkyl, C₁-C₆-haloalkyl, halogen or cyano.

2. Process for preparing compounds of the general formula (I) according to Claim 1, **characterized in that**
(A) anilines of the formula (II) 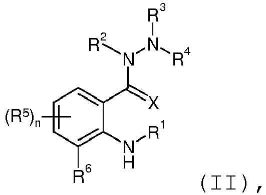 in which R¹, R², R³, R⁴, R⁵, R⁶, X and n are as defined in Claim 1,
are reacted with carbonyl chlorides of the formula (III) 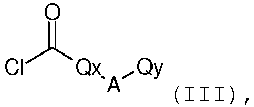 where
Qx, A and Qy are as defined in Claim 1,
in the presence of an acid-binding agent; or
(B) anilines of the formula (II) 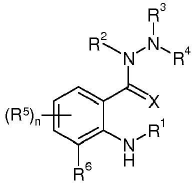 in which R¹, R², R³, R⁴, R⁵, R⁶, X and n are as defined in Claim 1,
are reacted with a carboxylic acid of the formula (IV) 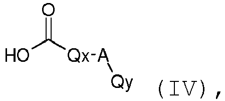 where
Qx, A and Qy are as defined in Claim 1,
in the presence of a condensing agent; or
(C) benzoxazinones of the formula (V) 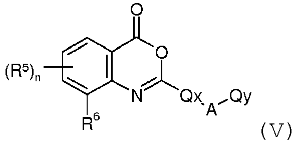 in which R⁵, R⁶, Qx, A, Qy and n are as defined in Claim 1,
are reacted with a hydrazine of the formula (VI) 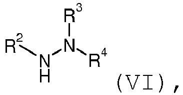 in which R², R³ and R⁴ are as defined in Claim 1,
in the presence of a diluent; or
(D) anthranilic hydrazides of the formula (VII) 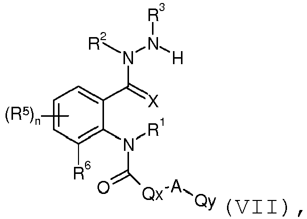 in which R¹, R², R³, R⁵, R⁶, X, n, Qx, A and Qy are as defined in Claim 1,
are reacted with a unit Y-R⁴, where R⁴ is as defined in Claim 1 and Y represents a suitable leaving group such as halogen or alkoxy; or
(E) anthranilic hydrazides of the formula (VII) 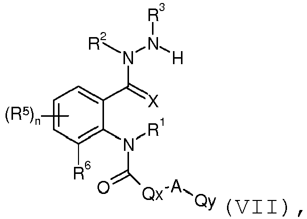 in which R¹, R², R³, R⁵, R⁶, X, n, Qx, A and Qy are as defined in Claim 1,
are reacted with an acid anhydride of the general formula (C(=O)-R⁸)₂O or (C(=O)-OR⁹)₂O
where R⁸ and R⁹ are as defined in Claim 1,
to give compounds of the formula (I) of the invention.

3. Mixtures of compounds of the general formula (I) according to Claim 1, in which Qy is Q62 and Q63, the ratio of a compound of the formula (I) in which Qy is Q62 to a compound of the formula (I) in which Qy is Q63 being 60:40 to 99:1.

4. Mixtures of compounds of the general formula (I) according to Claim 1, in which Qy is Q58 and Q59, the ratio of a compound of the formula (I) in which Qy is Q58 to a compound of the formula (I) in which Qy is Q59 being 60:40 to 99:1.

5. Compounds of the general formula (VII), 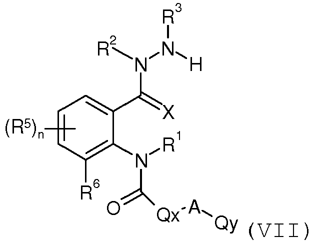 in which R¹, R², R³, R⁵, R⁶, X, n, Qx, A and Qy are as defined in Claim 1.

6. Agrochemical compositions comprising at least one compound of the formula (I) or a mixture of compounds of the formula (I) according to any of Claims 1, 3 or 4, and also extenders and/or surface-active substances.

7. Process for producing agrochemical compositions, **characterized in that** at least one compound of the general formula (I) or a mixture of compounds of the general formula (I) according to any of Claims 1, 3 or 4 is mixed with extenders and/or surface-active substances.

8. Use of a compound of the general formula (I) according to Claim 1, a mixture of compounds according to Claim 3 or 4 or a composition according to Claim 6 for controlling animal pests, with the exception of for the therapeutic treatment of the animal or human body.

9. Method for controlling animal pests, with the exception of for the therapeutic treatment of the animal or human body, **characterized in that** a compound of the general formula (I) according to Claim 1, a mixture of compounds of the general formula (I) according to Claim 3 or 4 or a composition according to Claim 6 is caused to act on animal pests and/or phytopathogenic fungi and/or their habitat and/or seed.

## Revendications

1. Dérivés d'acide anthranilique de formule générale (I) 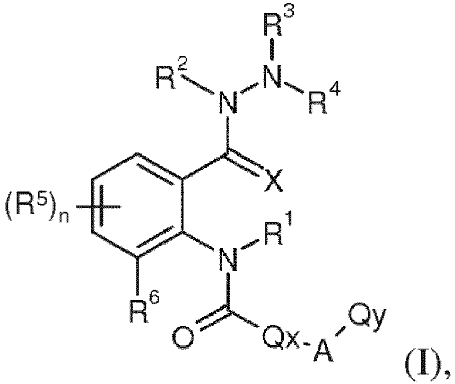 dans laquelle
R¹ représente un atome d'hydrogène,
R², R³ représentent indépendamment l'un de l'autre un atome d'hydrogène, le groupe méthyle, éthyle, isopropyle, tert-butyle,
R⁴ représente -C(=O)-R⁸, -C(=O)-OR⁹,
R⁵ représente un atome d'hydrogène, le groupe méthyle, trifluorométhyle, cyano, un atome de fluor, de chlore, de brome, d'iode ou le groupe trifluorométhoxy,
deux radicaux R⁵ contigus représentent également -(CH=CH-)₂,
n représente 2,
X représente O,
R⁶ représente le groupe méthyle ou un atome de chlore,
Qₓ représente un groupe 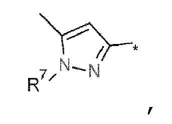
qui est une fois substitué par le groupe R⁷ 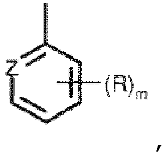 et où * désigne la liaison à A,
A représente CH₂, CH(CH₃) ou -CH₂O-,
R représente chaque fois indépendamment un atome de fluor, chlore ou brome,
m représente 1,
Z représente N, CCl ou CH,
Q_{y} représente un cycle hétéroaromatique de la série Q-58, Q-59, Q-62, Q-63, 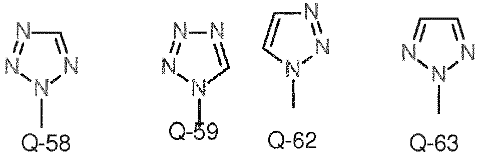 portant un ou plusieurs substituants, identiques ou différents, les substituants pouvant être choisis, indépendamment les uns des autres, parmi méthyle, éthyle, cyclopropyle, tert-butyle, chloro, fluoro, iodo, bromo, cyano, nitro, difluorométhyle, trifluorométhyle, pentafluoroéthyle, n-heptafluoropropyle ou iso-heptafluoropropyle ou les substituants pouvant être choisis, indépendamment les uns des autres, parmi phényle ou un cycle hétéroaromatique à 5 ou 6 chaînons, sur lesquels les substituants peuvent être choisis, indépendamment les uns des autres, parmi méthyle, éthyle, cyclopropyle, tert-butyle, chloro, fluoro, iodo, bromo, cyano, nitro, difluorométhyle, trifluorométhyle, pentafluoroéthyle, n-heptafluoropropyle ou iso-heptafluoropropyle,
R⁸ représente un atome d'hydrogène, un groupe méthyle, éthyle, isopropyle, tert-butyle, portant éventuellement un ou plusieurs substituants identiques ou différents, les substituants pouvant être choisis indépendamment les uns des autres parmi halogéno, cyano, phényle ou pyridyle, le groupe phényle ou pyridyle portant éventuellement un ou plusieurs substituants, identiques ou différents, choisis parmi hydrogéno, trifluorométhyle, cyano, fluoro, chloro, bromo ou trifluorométhoxy,
R⁸ représente en outre un groupe phényle, pyridyle ou un hétérocycle saturé à 3 à 6 chaînons, contenant 1-2 hétéroatomes choisis dans la série N, S, O, le cycle phényle ou pyridyle ou l'hétérocycle portant éventuellement un ou plusieurs substituants identiques ou différents, et les substituants pouvant être choisis, indépendamment les uns des autres, parmi hydrogéno, alkyle en C₁-C₆, halogéno-alkyle(C₁-C₆), halogéno ou cyano,
R⁹ représente un groupe méthyle, éthyle, isopropyle ou tert-butyle, portant éventuellement un ou plusieurs substituants identiques ou différents, les substituants pouvant être choisis indépendamment les uns des autres parmi halogéno, cyano, phényle ou pyridyle, le groupe phényle ou pyridyle portant éventuellement un ou plusieurs substituants, identiques ou différents, choisis parmi trifluorométhyle, cyano, fluoro, chloro, bromo ou trifluorométhoxy,
R⁹ représente en outre un groupe phényle, pyridyle ou un hétérocycle saturé à 3 à 6 chaînons, contenant 1-2 hétéroatomes choisis dans la série N, S, O, le cycle phényle ou pyridyle ou l'hétérocycle portant éventuellement un ou plusieurs substituants identiques ou différents, et les substituants pouvant être choisis, indépendamment les uns des autres, parmi hydrogéno, alkyle en C₁-C₆, halogénoalkyle(C₁-C₆), halogéno ou cyano.

2. Procédé pour la préparation de composés de formule générale (I) selon la revendication 1, **caractérisé en ce qu'**on fait réagir
(A) des anilines de formule (II) 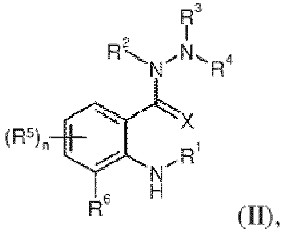 dans laquelle R¹, R², R³, R⁴, R⁵, R⁶, X et n ont les significations selon la revendication 1,
avec des chlorures d'acides carboxyliques de formule (III) 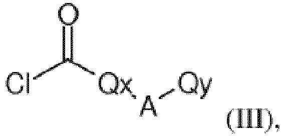 dans laquelle
Qx, A et Qy ont les significations selon la revendication 1,
en présence d'un capteur d'acide ; ou
(B) des anilines de formule (II) 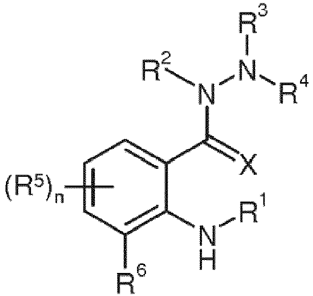 dans laquelle R¹, R², R³, R⁴, R⁵, R⁶, X et n ont les significations selon la revendication 1,
avec un acide carboxylique de formule (IV) 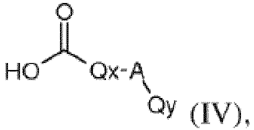 dans laquelle
Qx, A et Qy ont les significations selon la revendication 1,
en présence d'un agent de condensation ; ou
(C) des benzoxazinones de formule (V) 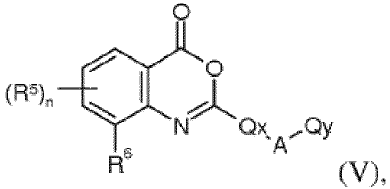 dans laquelle R⁵, R⁶, Qx, A, Qy et n ont les significations selon la revendication 1,
avec une hydrazine de formule (VI) 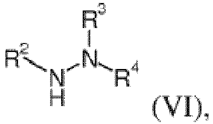 dans laquelle R², R³, R⁴ ont les significations selon la revendication 1,
en présence d'un diluant ; ou
(D) des hydrazides d'acide anthranilique de formule (VII) 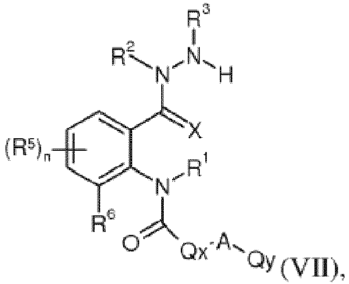 dans laquelle R¹, R², R³, R⁵, R⁶, X, n, Qx, A, Qy ont les significations selon la revendication 1,
avec un composant Y-R⁴, où R⁴ a la signification selon la revendication 1 et Y représente un groupe partant convenable, tel qu'halogéno ou alcoxy ; ou
(E) des hydrazides d'acide anthranilique de formule (VII) 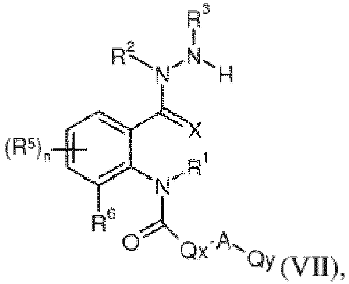 dans laquelle R¹, R², R³, R⁵, R⁶, X, n, Qx, A, Qy ont les significations selon la revendication 1,
avec un anhydride d'acide de formule générale (C(=O)-R⁸)₂O ou (C(=O)-OR⁹)₂O
R⁸, R⁹ ayant les significations selon la revendication 1,
pour aboutir aux composés de formule (I) selon l'invention.

3. Mélanges de composés de formule générale (I) selon la revendication 1, dans lesquels Qy représente Q62 et Q63, le rapport d'un composé de formule (I), dans lequel Qy représente Q62, à un composé de formule (I), dans lequel Qy représente Q63, valant de 60:40 à 99:1.

4. Mélanges de composés de formule générale (I) selon la revendication 1, dans lesquels Qy représente Q58 et Q59, le rapport d'un composé de formule (I), dans lequel Qy représente Q58, à un composé de formule (I), dans lequel Qy représente Q59, valant de 60:40 à 99:1.

5. Composés de formule générale (VII), 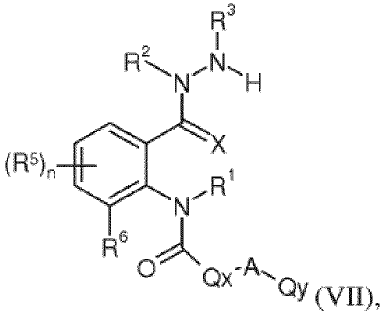 dans laquelle R¹, R², R³, R⁵, R⁶, X, n, Qx, A, Qy ont les significations selon la revendication 1.

6. Compositions agrochimiques contenant au moins un composé de formule (I) ou un mélange de composés de formule (I) selon l'une quelconque des revendications 1, 3 ou 4, ainsi que des excipients et/ou des substances tensioactives.

7. Procédé pour la préparation de compositions agrochimiques, **caractérisé en ce qu'**on mélange au moins un composé de formule générale (I) ou un mélange de composés de formule générale (I) selon l'une quelconque des revendications 1, 3 ou 4, avec des excipients et/ou des substances tensioactives.

8. Utilisation d'un composé de formule générale (I) selon la revendication 1, d'un mélange de composés selon la revendication 3 ou 4 ou d'une composition selon la revendication 6, pour la lutte contre des ravageurs animaux, à l'exclusion du traitement thérapeutique de l'organisme humain ou animal.

9. Procédé pour la lutte contre des ravageurs animaux - à l'exclusion du traitement thérapeutique de l'organisme humain ou animal - **caractérisé en ce qu'**on fait agir sur des ravageurs animaux et/ou des champignons phytopathogènes et/ou leur habitat et/ou des semences un composé de formule générale (I) selon la revendication 1, un mélange de composés de formule générale (I) selon la revendication 3 ou 4 ou une composition selon la revendication 6.
